# FASCICULE DE BREVET EUROPEEN

(11) **EP 0 912 485 B1**
(45) Date de publication et mention de la délivrance du brevet: **31.10.2001**
(21) Numéro de dépôt: 97926057.7
(22) Date de dépôt: 29.05.1997
(51) Int. Cl.: C07C 55/14, C07C 51/14

(54) **PROCEDE D'HYDROXYCARBONYLATION DES ACIDES PENTENOIQUES**
CARBONYLIERUNGSVERFAHREN DER PENTENSÄUREN
PENTENOIC ACID HYDROXYCARBONYLATION METHOD

(30) Priorité: 07.06.1996 FR 9607380
(43) Date de publication de la demande: 06.05.1999
(73) Titulaire: RHODIA POLYAMIDE INTERMEDIATES, 69190 Saint-Fons (FR)
(72) Inventeur: BRIVET, Jacques, F-69500 Bron (FR); HENRIET, Eric, B., F-69003 Lyon (FR); PATOIS, Carl, F-69003 Lyon (FR); PERRON, Robert, F-69390 Charly (FR)
(74) Mandataire: Esson, Jean-Pierre
(86) Numéro de dépôt international: FR9700940
(87) Numéro de publication internationale: WO9747580

(56) Documents cités:
- US-A- 5 218 144

## Description

La présente invention concerne l'hydroxycarbonylation des acides penténoïques en acide adipique.

Lors de l'hydroxycarbonylation, en présence d'un catalyseur et d'un promoteur, des acides penténoïques, principalement de l'acide 3-penténoïque, en acide adipique, il se forme également des quantités minoritaires, mais néanmoins importantes, de diacides carboxyliques ramifiés, isomères de l'acide adipique, essentiellement l'acide méthyl-2 glutarique et l'acide éthyl-2 succinique, ainsi que des traces d'acide diméthyl-2,2 succinique.

Après séparation de l'acide adipique, l'acide penténoïque non transformé, le catalyseur, le promoteur et divers sous-produits tel que la gamma-valérolactone, sont recyclés dans le réacteur d'hydroxycarbonylation.

Cependant, alors qu'il est avantageux au plan de l'économie du procédé de recycler au mieux l'acide penténoïque non transformé, le catalyseur, le promoteur et les sous-produits susceptibles d'être transformés au moins partiellement en acide adipique, la Demanderesse a constaté que le recyclage de quantités trop importantes de diacides carboxyliques ramifiés a une influence néfaste sur l'activité du catalyseur dans la réaction d'hydroxycarbonylation de l'acide penténoïque.

Outre cet effet de désactivation du catalyseur, la présence de diacides ramifiés nuit bien évidemment à la pureté de l'acide adipique, notamment par le piégeage du catalyseur métallique lors de la cristallisation dudit acide adipique.

La présente invention concerne donc plus particulièrement un procédé d'hydroxycarbonylation d'acide penténoïque par réaction avec l'eau et le monoxyde de carbone, en présence d'un catalyseur comportant au moins du rhodium et/ou de l'iridium et d'un promoteur iodé ou bromé, procédé dans lequel le catalyseur provient au moins en partie d'une opération antérieure d'hydroxycarbonylation d'acide penténoïque, caractérisé en ce que la réaction est effectuée en présence d'une quantité de diacides carboxyliques ramifiés ayant 6 atomes de carbone inférieure ou égale à 200 grammes par kilogramme de mélange réactionnel.

Les termes diacides carboxyliques ramifiés ou diacides ramifiés sont équivalents dans le présent texte et couvrent également les formes anhydrides correspondant à ces diacides.

De préférence, dans le procédé selon l'invention la réaction d'hydroxycarbonylation est conduite en présence d'une quantité de diacides carboxyliques ramifiés ayant 6 atomes de carbone inférieure ou égale à 150 grammes par kilogramme de mélange réactionnel

L'hydroxycarbonylation de l'acide penténoïque est effectuée en présence d'un catalyseur comprenant du rhodium et/ou de l'iridium, et éventuellement d'autres métaux nobles choisis parmi le ruthénium et l'osmium. La quantité de catalyseur à mettre en oeuvre peut varier dans de larges limites.

En général, une quantité exprimée en moles d'iridium métallique et/ou de rhodium métallique par litre de mélange réactionnel comprise entre 10⁻⁴ et 10⁻¹ conduit à des résultats satisfaisants. Des quantités inférieures peuvent être mises en oeuvre: on observe toutefois que la vitesse de réaction est faible. Des quantités supérieures n'ont d'inconvénients qu'au plan économique.

De préférence la concentration en iridium et/ou rhodium est comprise entre 5.10⁻⁴ et 10⁻² mole/litre.

Par promoteur iodé ou bromé, on entend dans le cadre du procédé de l'invention Hl et HBr et les composés organo-iodés ou organo-bromés capables de générer Hl ou HBr dans les conditions réactionnelles et plus particulièrement les iodures et bromures d'alkyles ayant 1 à 10 atomes de carbone. L'iodure de méthyle et le bromure de méthyle sont plus particulièrement préconisés.

De préférence le promoteur utilisé est un promoteur iodé et encore préférentiellement Hl et l'iodure de méthyle.

La quantité de promoteur iodé et/ou bromé à mettre en oeuvre est généralement telle que le rapport molaire iode (et/ou brome)/Iridium(et/ou Rhodium) est supérieur ou égal à 0,1. Il est généralement préférable que ce rapport soit inférieur ou égal à 20. De préférence le rapport molaire iode (et/ou brome)/lridium(et/ou Rhodium) est compris entre 1 et 5.

La présence d'eau est indispensable à la conduite de l'hydroxycarbonylation. Généralement la quantité d'eau à mettre en oeuvre est telle que le rapport molaire eau/acides penténoïques est compris entre 0,01 et 10.

Une quantité supérieure n'est pas souhaitable en raison de la perte d'activité catalytique observée. Le rapport molaire eau/acides penténoïques dans le mélange réactionnel peut être instantanément inférieur à la valeur minimale indiquée précédemment, si l'on procède par exemple à l'injection continue de l'eau, plutôt qu'à son introduction avec les autres charges avant la réaction d'hydroxycarbonylation.

De préférence, le rapport molaire eau/acides penténoïques est compris entre 0,01 et 2, la remarque précédente concernant la valeur minimale étant également valable.

La réaction d'hydroxycarbonylation peut être conduite soit dans un tiers solvant, soit dans un fort excès d'acides penténoïques.

Comme tiers solvant, on peut utiliser notamment les acides carboxyliques aliphatiques saturés ou aromatiques comportant au maximum 20 atomes de carbone pour autant qu'ils soient liquides dans les conditions réactionnelles. A titre d'exemples de tels acides carboxyliques, on peut citer l'acide acétique, l'acide propionique, l'acide butyrique, l'acide valérique, l'acide adipique, l'acide benzoïque et l'acide phénylacétique.

Le tiers solvant peut également être choisi parmi les hydrocarbures saturés aliphatiques ou cycloaliphatiques et leurs dérivés chlorés et les hydrocarbures aromatiques et leurs dérivés chlorés, pour autant que ces composés soient liquides dans les conditions réactionnelles. A titre d'exemples de tels solvants, on peut citer le benzène, le toluène, le chlorobenzène, le dichlorométhane, l'hexane et le cyclohexane.

Lorsqu'il est présent dans le mélange réactionnel, le tiers solvant représente par exemple de 10 % à 99 % en volume par rapport au volume total dudit mélange réactionnel et de préférence de 30 % à 90 % en volume.

Selon une variante préférentielle, la réaction d'hydroxycarbonylation est réalisée dans les acides penténoïques eux-mêmes, c'est-à-dire l'acide pentène-2-oïque, l'acide pentène-3-oïque, l'acide pentène-4-oïque et leurs mélanges.

La réaction d'hydroxycarbonylation est conduite sous une pression supérieure à la pression atmosphérique et en présence de monoxyde de carbone. On peut utiliser le monoxyde de carbone sensiblement pur ou de qualité technique tel qu'il se trouve dans le commerce.

La réaction est conduite en phase liquide. La température est généralement comprise entre 100°C et 240°C et de préférence entre 160°C et 200°C.

La pression totale peut varier dans de larges limites. La pression partielle du monoxyde de carbone, mesurée à 25°C, est généralement de 0,5 bar à 50 bars et de préférence de 1 bar à 25 bars.

Comme indiqué précédemment, le mélange réactionnel issu de la réaction d'hydroxycarbonylation comporte essentiellement les acides penténoïques non transformés, l'eau, le promoteur iodé et/ou bromé, le catalyseur, le solvant éventuellement mis en oeuvre, l'acide adipique obtenu, les autrcs sous-produits formés en quantités plus ou moins importantes, comme par exemple l'acide méthyl-2 glutarique, l'acide éthyl-2 succinique, l'acide valérique ou la gamma-valérolactone (ou méthyl-4 butyrolactone).

La séparation au moins partielle des diacides ramifiés, afin que leur concentration après recyclage dans le réacteur d'hydroxycarbonylation ne dépasse pas les limites supérieures indiquées précédemment, tout au long de la réaction, peut se faire selon des techniques connues. On peut par exemple transformer tout ou partie des diacides ramifiés en leurs anhydrides correspondants, comme décrit dans le brevet EP-A-0 687 663, afin de pouvoir les séparer plus facilement par distillation.

On peut également opérer une distillation fractionnée sur le mélange réactionnel issu de l'hydroxycarbonylation et éliminer les composés les plus légers tels que les acides penténoïques ou les autres composés à 5 atomes de carbone. Cette première distillation peut être effectuée sous pression atmosphérique, éventuellement après séparation par cristallisation d'une partie de l'acide adipique formé. Ensuite on peut distiller les fractions les plus riches en diacides ramifiés. Cette distillation peut être complétée par d'autres opérations de séparation des divers constituants du mélange réactionnel issu de l'hydroxycarbonylation. Ainsi on peut réaliser la cristallisation et éventuellement une ou plusieurs recristallisations de l'acide adipique pour récupérer le maximum du catalyseur qu'il contient.

La distillation des diacides ramifiés est effectuée sous pression réduite et peut avantageusement être conduite sous un léger balayage de monoxyde de carbone.

On peut ainsi recycler au moins une partie du catalyseur, du promoteur et des composés légers susceptibles d'être au moins partiellement transformés en acide adipique, tout en ne recyclant que très peu ou pas de diacides ramifiés.

De manière avantageuse, le procédé de l'invention est mis en oeuvre de manière continue. En effet, outre l'intérêt industriel évident de ce type de réalisation; il est beaucoup plus facile de maintenir en permanence un taux de diacides ramifiés relativement faible, la formation inévitable desdits diacides ramifiés et leur recyclage partiel étant compensés par le soutirage continu d'une partie du mélange réactionnel.

La concentration du mélange réactionnel en diacides ramifiés peut alors être maintenue par exemple à une valeur inférieure ou égale à 100 grammes par kilogramme et de préférence inférieure ou égale à 50 grammes par kilogramme.

Enfin parmi les diacides ramifiés, il s'avère qu'il est encore plus avantageux que l'acide éthyl-2 succinique soit plus spécifiquement maintenu dans le milieu réactionnel à une teneur inférieure ou égale à 50 grammes par kilogramme et de préférence inférieure ou égale à 30 grammes par kilogramme. Dans le cadre d'un procédé en marche continue, cette teneur en acide éthyl-2 succinique peut être alors maintenue dans le milieu réactionnel à une valeur inférieure ou égale à 20 grammes par kilogramme et de préférence inférieure ou égale à 10 grammes par kilogramme.

Les exemples qui suivent illustrent l'invention.

### EXEMPLES 1 A 3

Dans un réacteur métallique de 1 litre, muni de moyens de chauffage et de refroidissement, d'une agitation (à 1200 tours/minute), de dispositifs d'introduction des réactifs et de soutirage, d'appareils de mesure de la température et de la pression, on charge :
- 2,52 mol d'acide 3-penténoïque (P3)
- 0,924 mmol de Ir Cl(COD)
- 2,24 mmol de Hl (solution aqueuse à 57 % en poids)

On établit une pression de 5 bar de CO à température ambiante, puis on chauffe sous agitation à 185°C, température à laquelle la pression est ajustée à 20 bar à l'aide de CO. On injecte alors 22,7 g d'eau (1,26 mol) en 30 minutes.

Après 30 min de réaction, le mélange réactionnel est soutiré à chaud, sous atmosphère de monoxyde de carbone dans une chaudière de 500 ml.

Un échantillon de ce mélange est dosé par chromatographie en phase gazeuse (CPG) et en chromatographie liquide à haute performance (CLHP).

On trouve les résultats suivants :
- taux de transformation (TT) de P3 : 52 %
- rendement (RT) en acide adipique (AdOH) par rapport à P3 transformé : 68 %
- rendement (RT) en diacides ramifiés (acide méthyl-2 glutarique et éthyl-2 succinique par rapport à P3 transformé : 13 %
- rendement (RT) en gamma valérolactone (M4L) par rapport à P3 transformé : 8 %
- vitesse d'absorption de CO en moles par heure et par litre de mélange réactionnel (environ 280 ml) : 5,8
- linéarité (L) (= rapport acide adipique /ensemble des diacides obtenus) : 84 %

Le mélange soutiré est distillé à l'aide d'une colonne de 250 cm de hauteur, sous pression atmosphérique, avec un bullage de CO.

On obtient une fraction contenant l'acide penténoïque non transformé, la gamma-valérolactone, les acides valérique et méthylbutanoïque ainsi qu'une partie des acides méthyl-glutarique et éthyl-succinique.

On distille ensuite les acides méthyl-glutarique et éthyl-succinique sous pression réduite (avec un bullage de CO par l'intermédiaire d'un tube capillaire).

Le résidu contient essentiellement l'acide adipique et le catalyseur. Le catalyseur est récupéré dans les eaux de recristallisation et de lavage de l'acide acipique.

Après avoir complété la quantité de catalyseur, rajouté le promoteur iodé et de l'acide 3-penténoïque, on répète l'hydroxycarbonylation de l'acide penténoïque comme décrit précédemment, avec sensiblement les mêmes quantités de réactifs et dans les mêmes conditions opératoires, mais en recyclant une partie des diacides ramifiés isolés précédemment.

Les charges sont les suivantes :
- 2,44 mol d'acide 3-penténoïque (P3)
- 0,924 mmol de Ir Cl(COD)
- 2,24 mmol de Hl (solution aqueuse à 57 % en poids)
- 11,5 g de diacides ramifiés (41 g/kg de mélange réactionnel initial)
- 1,26 mol d'eau (22,7 g) injectée en 30 minutes.

La réaction dure 30 min à 185°C et après dosage on trouve les résultats suivants :
- taux de transformation (TT) de P3 : 53 %
- rendement (RT) en acide adipique (AdOH) par rapport à P3 transformé : 66 %
- rendement (RT) en diacides ramifiés (acide méthyl-2 glutarique et éthyl-2 succinique par rapport à P3 transformé : 15,5 %
- rendement (RT) en gamma valérolactone (M4L) par rapport à P3 transformé : 8 %
- vitesse d'absorption de CO en moles par heure et par litre de mélange réactionnel (environ 280 ml) : 5,8
- linéarité (L) (= rapport acide adipique /ensemble des diacides obtenus) : 81 %
- teneur totale en diacides ramifiés dans le mélange réactionnel final : 134 g/kg.

On effectue le traitement décrit pour l'exemple 1 et après avoir complété la quantité de catalyseur, rajouté le promoteur iodé et de l'acide 3-penténoïque, on répète l'hydroxycarbonylation de l'acide penténoïque comme décrit précédemment, avec sensiblement les mêmes quantités de réactifs et dans les mêmes conditions opératoires, mais en recyclant une quantité plus importante des diacides ramifiés isolés précédemment.

Les charges sont les suivantes :
- 2,35 mol d'acide 3-penténoïque (P3)
- 0,924 mmol de Ir Cl(COD)
- 2,24 mmol de Hl (solution aqueuse à 57 % en poids)
- 22,9 g de diacides ramifiés (41,5 g/kg de mélange réactionnel initial)
- 1,26 mol d'eau (22,7 g) injectée en 30 minutes.

La réaction dure 30 min à 185°C et après dosage on trouve les résultats suivants :
- taux de transformation (TT) de P3 : 49 %
- rendement (RT) en acide adipique (AdOH) par rapport à P3 transformé : 65 %
- rendement (RT) en diacides ramifiés (acide méthyl-2 glutarique et éthyl-2 succinique par rapport à P3 transformé : 18,3 %
- rendement (RT) en gamma valérolactone (M4L) par rapport à P3 transformé : 7 %
- vitesse d'absorption de CO en moles par heure et par litre de mélange réactionnel (environ 280 ml) : 5,2
- linéarité (L) (= rapport acide adipique /ensemble des diacides obtenus) : 78 %
- teneur totale en diacides ramifiés dans le mélange réactionnel final : 177 g/kg.

On observe dans les résultats obtenus dans l'exemple 3, avec une teneur en diacides ramifiés comprise entre 81,5 g/kg au début de l'hydroxycarbonylation et 177g/kg à la fin de l'hydroxycarbonylation, un début de diminution de la vitesse de réaction et de la linéarité.

### ESSAI COMPARATIF 1

On répète les essais de recyclage en introduisant des quantités plus importantes d'acide méthyl-glutarique et d'acide éthyl-succinique, les conditions opératoires étant les mêmes.

Les charges sont les suivantes :
- 2,21-mol d'acide 3-penténoïque (P3)
- 0,924 mmol de Ir Cl(COD)
- 2,24 mmol de HI (solution aqueuse à 57 % en poids)
- 45,8 g de diacides ramifiés (158 g/kg de mélange réactionnel initial)
- 1,26 mol d'eau (22,7 g) injectée en 30 minutes.

La réaction dure 30 min à 185°C et après dosage on trouve les résultats suivants :
- taux de transformation (TT) de P3 : 29%
- rendement (RT) en acide adipique (AdOH) par rapport à P3 transformé : 53 %
- rendement (RT) en diacides ramifiés (acide méthyl-2 glutarique et éthyl-2 succinique par rapport à P3 transformé : 31,1 %
- rendement (RT) en gamma valérolactone (M4L) par rapport à P3 transformé : 4 %
- vitesse d'absorption de CO en moles par heure et par litre de mélange réactionnel (environ 280 ml) : 2,8
- linéarité (L) (= rapport acide adipique /ensemble des diacides obtenus) : 63 %
- teneur totale en diacides ramifiés dans le mélange réactionnel final : 257 g/kg.

On observe dans les résultats obtenus dans l'essai comparatif 1, avec une teneur en diacides ramifiés comprise entre 158 g/kg au début de l'hydroxycarbonylation et 257g/kg à la fin de l'hydroxycarbonylation, une très forte diminution de la vitesse de réaction et de la linéarité des diacides obtenus.

## Revendications

1. Procédé d'hydroxycarbonylation d'acide penténoïque par réaction avec l'eau et le monoxyde de carbone, en présence d'un catalyseur comportant au moins du rhodium et/ou de l'iridium et d'un promoteur iodé ou bromé, procédé dans lequel au moins une partie de l'acide penténoïque, du catalyseur et du promoteur proviennent d'une opération antérieure d'hydroxycarbonylation d'acide penténoïque, **caractérisé en ce que** la réaction est effectuée en présence d'une quantité de diacides carboxyliques ramifiés ayant 6 atomes de carbone inférieure ou égale à 200 grammes par kilogramme de mélange réactionnel.

2. Procédé selon la revendication 1, **caractérisé en ce que** la réaction d'hydroxycarbonylation est conduite en présence d'une quantité de diacides carboxyliques ramifiés ayant 6 atomes de carbone inférieure ou égale à 150 grammes par kilogramme de mélange réactionnel.

3. Procédé selon l'une des revendications 1 ou 2, **caractérisé en ce que** la réaction d'hydroxycarbonylation est effectuée en présence d'un catalyseur comprenant du rhodium et/ou de l'iridium, et éventuellement d'autres métaux nobles choisis parmi le ruthénium et l'osmium.

4. Procédé selon l'une des revendications 1 à 3, **caractérisé en ce que** la quantité de catalyseur mise en oeuvre est comprise entre 10⁻⁴ et 10⁻¹ en moles d'iridium métallique et/ou de rhodium métallique par litre de mélange réactionnel et de préférence est comprise entre 5.10⁻⁴ et 10⁻² mole/litre.

5. Procédé selon l'une des revendications 1 à 4, **caractérisé en ce que** le promoteur iodé ou bromé est choisi parmi Hl et HBr et les composés organo-iodés ou organo-bromés capables de générer HI ou HBr dans les conditions réactionnelles, tels que les iodures et bromures d'alkyles ayant 1 à 10 atomes de carbone.

6. Procédé selon l'une des revendications 1 à 5, **caractérisé en ce que** le promoteur utilisé est un promoteur iodé et de préférence Hl ou l'iodure de méthyle.

7. Procédé selon l'une des revendications 1 à 6, **caractérisé en ce que** la quantité de promoteur iodé et/ou bromé mise en oeuvre est telle que le rapport molaire iode( et/ou brome/lridium(et/ou Rhodium) est supérieur ou égal à 0,1 et de préférence inférieur ou égal à 20.

8. Procédé selon l'une des revendications 1 à 7, **caractérisé en ce que** la quantité d'eau mise en oeuvre est telle que le rapport molaire eau/acides penténoïques est compris entre 0,01 et 10.

9. Procédé selon l'une des revendications 1 à 8, **caractérisé en ce que** la réaction d'hydroxycarbonylation est conduite soit dans un tiers solvant, soit dans un fort excès d'acides penténoïques.

10. Procédé selon la revendication 9, **caractérisé en ce que** le tiers solvant est choisi parmi les acides carboxyliques aliphatiques saturés ou aromatiques comportant au maximum 20 atomes de carbone ou parmi les hydrocarbures saturés aliphatiques ou cycloaliphatiques et leurs dérivés chlorés et les hydrocarbures aromatiques et leurs dérivés chlorés, pour autant que ces composés soient liquides dans les conditions réactionnelles.

11. Procédé selon l'une des revendications 1 à 10, **caractérisé en ce qu'**il est mis en oeuvre de manière continue et que la concentration du mélange réactionnel en diacides ramifiés est à une valeur inférieure ou égale à 100 grammes par kilogramme et de préférence inférieure ou égale à 50 grammes par kilogramme.

## Patentansprüche

1. Verfahren zur Hydroxycarbonylierung von Pentensäure durch Umsetzung mit Wasser und Kohlenmonoxid in Gegenwart eines zumindest Rhodium und/oder Iridium umfassenden Katalysators und eines jod- oder bromhaltigen Promotors, wobei in dem Verfahren zumindest ein Teil der Pentensäure, des Katalysators und des Promotors von einer vorangegangenen Hydroxycarbonylierungsmaßnahme der Pentensäure stammen, **dadurch gekennzeichnet, daß** die Reaktion in Gegenwart einer Menge an verzweigten Dicarbonsäuren mit 6 Kohlenstoffatomen von geringer als oder gleich 200 g je kg Reaktionsgemisch durchgeführt wird.

2. Verfahren gemäß Anspruch 1, **dadurch gekennzeichnet, daß** die Hydroxycarbonylierungsreaktion in Gegenwart einer Menge an verzweigten Dicarbonsäuren mit 6 Kohlenstoffatomen von geringer als oder gleich 150 g je kg Reaktionsgemisch durchgeführt wird.

3. Verfahren gemäß einem der Ansprüche 1 oder 2, **dadurch gekennzeichnet, daß** die Hydroxycarbonylierungsreaktion in Gegenwart eines Rhodium und/oder Iridium und gegebenenfalls weitere Edelmetalle, ausgewählt unter Ruthenium und Osmium, umfassenden Katalysators durchgeführt wird.

4. Verfahren gemäß einem der Ansprüche 1 bis 3, **dadurch gekennzeichnet, daß** die Menge des eingesetzten Katalysators zwischen 10⁻⁴ und 10⁻¹, ausgedrückt in Mol metallisches Iridium und/oder metallisches Rhodium je Liter Reaktionsgemisch, und vorzugsweise zwischen 5^{.} 10⁻⁴ und 10⁻² Mol/Liter beträgt.

5. Verfahren gemäß einem der Ansprüche 1 bis 4, **dadurch gekennzeichnet, daß** der jod- oder bromhaltige Promotor ausgewählt wird unter HJ und HBr und den Jod-Organo-Verbindungen oder Brom-Organo-Verbindungen, die imstande sind HJ oder HBr unter den Reaktionsbedingungen zu entwickeln, wie den Akyljodiden und -bromiden mit 1 bis 10 Kohlenstoffatomen.

6. Verfahren gemäß einem der Ansprüche 1 bis 5, **dadurch gekennzeichnet, daß** der verwendete Promotor ein jodhaltiger Promotor und vorzugsweise HI oder Methyljodid ist.

7. Verfahren gemäß einem der Ansprüche 1 bis 6, dadurch gckennzeicänet, daß die Menge des eingesetzten jod- und/oder bromhaltigen Promotors derart ist, daß das Molverhältnis Jod (und/oder Brom)/Iridium (und/oder Rhodium) höher als oder gleich 0,1 und vorzugsweise niedriger als oder gleich 20 ist.

8. Verfahren gemäß einem der Ansprüche 1 bis 7, **dadurch gekennzeichnet, daß** die Menge an eingesetztem Wasser derart ist, daß das Molverhältnis Wasser/Pentensäuren zwischen 0,01 und 10 liegt.

9. Verfahren gemäß einem der Ansprüche 1 bis 8, **dadurch gekennzeichnet, daß** die Hydroxycarbonylierungsreaktion entweder in einem dritten Lösungsmittel oder in einem starken Überschuß an Pentensäuren durchgeführt wird.

10. Verfahren gemäß Anspruch 9, **dadurch gekennzeichnet, daß** das dritte Lösungsmittel ausgewählt wird unter den gesättigten aliphatischen oder den aromatischen Carbonsäuren mit maximal 20 Kohlenstoffatomen oder den aliphatischen oder cycloaliphatischen gesättigten Kohlenwasserstoffen und deren chlorhaltigen Derivaten und den aromatischen Kohlenwasserstoffen und deren chlorhaltigen Derivaten, insoweit, als diese Verbindungen unter den Reaktionsbedingungen flussig sind.

11. Verfahren gemäß einem der Ansprüche 1 bis 10, **dadurch gekennzeichnet, daß** es in kontinuierlicher Weise durchgeführt wird und daß die Konzentration des Reaktionsgemisches an verzweigten Disäuren bei einem Wert von geringer als oder gleich 100 g je kg, und vorzugsweise geringer als oder gleich 50 g je kg, liegt.

## Claims

1. Process for the hydroxycarbonylation of pentenoic acid by reaction with water and carbon monoxide in the presence of a catalyst containing at least rhodium and/or iridium and of an iodinated or brominated promoter, in which process the catalyst originates at least in part from a prior operation for the hydroxycarbonylation of pentenoic acid, **characterized in that** the reaction is carried out in the presence of an amount of branched dicarboxylic acids having 6 carbon atoms of less than or equal to 200 grams per kilogram of reaction mixture.

2. Process according to claim 1, **characterized in that** the hydroxycarbonylation reaction is conducted in the presence of an amount of branched dicarboxylic acids having 6 carbon atoms of less than or equal to 150 grams per kilogram of reaction mixture.

3. Process according to one of claims 1 and 2, **characterized in that** the hydroxycarbonylation reaction is carried out in the presence of a catalyst comprising rhodium and/or iridium and optionally other noble metals chosen from ruthenium and osmium.

4. Process according to one of claims 1 to 3, **characterized in that** the amount of catalyst employed is between 10⁻⁴ and 10⁻¹ in moles of metallic iridium and/or of metallic rhodium per litre of reaction mixture and is preferably between 5 × 10⁻⁴ and 10⁻² mol/litre.

5. Process according to one of claims 1 to 4, **characterized in that** the iodinated or brominated promoter is chosen from HI and HBr and organoiodinated or organobrominated compounds capable of generating HI or HBr under the reaction conditions, such as alkyl iodides and bromides having 1 to 10 carbon atoms.

6. Process according to one of claims 1 to 5, **characterized in that** the promoter used is an iodinated promoter and preferably HI or methyl iodide.

7. Process according to one of claims 1 to 6, **characterized in that** the amount of iodinated and/or brominated promoter employed is such that the iodine (and/or bromine)/iridium (and/or rhodium) molar ratio is greater than or equal to 0.1 and preferably less than or equal to 20.

8. Process according to one of claims 1 to 7, **characterized in that** the amount of water employed is such that the water/pentenoic acids molar ratio is between 0.01 and 10.

9. Process according to one of claims 1 to 8, **characterized in that** the hydroxycarbonylation reaction is carried out either in a third solvent or in a large excess of pentenoic acids.

10. Process according to claim 9, **characterized in that** the third solvent is chosen from saturated aliphatic or aromatic carboxylic acids containing at most 20 carbon atoms or from saturated aliphatic or cycloaliphatic hydrocarbons and their chlorinated derivatives and aromatic hydrocarbons and their chlorinated derivatives, provided that these compounds are liquid under the reaction conditions.

11. Process according to one of claims 1 to 10, **characterized in that** it is implemented continuously and that the concentration of branched diacids in the reaction mixture is at a value of less than or equal to 100 grams per kilogram and preferably of less than or equal to 50 grams per kilogram.
